# EUROPEAN PATENT APPLICATION

(11) **EP 1 915 998 A1**
(43) Date of publication of application: **30.04.2008**
(21) Application number: 06767051.3
(22) Date of filing: 21.06.2006
(51) Int. Cl.: A61K 38/16, A61K 9/06, A61K 9/08, A61P 27/02

(54) **PROPHYLACTIC OR THERAPEUTIC AGENT FOR CORNEAL/CONJUNCTIVAL DISEASE**

(30) Priority: 22.06.2005 JP 2005182597
(71) Applicant: Kowa Co., Ltd., Nagoya-shi, Aichi 460-8625 (JP); Juridical Foundation The Chemo-Sero-Therapeutic Research Institute, Kumamoto-shi Kumamoto-ken 860-8568 (JP); Tsubota, Kazuo, Funabashi-shi, Chiba-ken (JP)
(72) Inventor: WATANABE, Masanao, 1890022 (JP); TSUBOTA, Kazuo, 2730031 (JP); HIRASHIMA, Masaki c/o KIKUCHI RESEARCH CENTER,, Kyokushi-kawabe, Kikuchi-shi, Kumamoto 8691298 (JP); NOZAKI, Chikateru c/o KIKUCHI RESEARCH CENTER,, Kyokushi-kawabe, Kikuchi-shi, Kumamoto 8691298 (JP)
(74) Representative: Albrecht, Thomas
(86) International application number: PCT/JP2006/312392
(87) International publication number: WO 2006/137426

(57) **Abstract**

Disclosed is a novel composition for the treatment of a corneal/conjunctival disease. A prophylactic or therapeutic agent for a corneal/conjunctival disease comprising selenoprotein P as an active ingredient, more specifically a prophylactic or therapeutic agent for a corneal/conjunctival disease such as dry eye, keratoconjunctivitis sicca, superficial punctate keratopathy, corneal erosion or corneal ulcer comprising selenoprotein P as an active ingredient, particularly a prophylactic or therapeutic agent for a corneal/conjuncrtival disease such as dry eye, keratoconjunctivitis sicca, superficial punctate keratopathy, corneal erosion or corneal ulcer accompanied by a corneal/conjunctival epithelial discorder.

## Description

### Technical Field

The present invention relates to a prophylactic or therapeutic agent for a corneal/conjunctival disease containing selenoprotein P as an active ingredient, particularly for such as dry eye, keratoconjunctivitis sicca, superficial punctate keratopathy, corneal erosion and corneal ulcer.

### Background Art

The cornea is the transparent and thin tissue of about 1 mm in thickness containing no blood vessels, and has an extremely regular, fine structure consisting of the corneal epithelium, Bowman's layer, stroma, Descemet's membrane and corneal endothelium. The cornea is the highly differentiated transparent and refractive tissue positioned at the front surface of an eyeball and allowing light to enter inside to a photoreceptor located in the retina. The cornea plays a particularly important role in vision. The cornea, being directly in contact with the outside environment, also functions as a barrier against the chemical invasions as well as the biological invasions of such as microorganisms. The corneal epithelium is covered with the tear film, which keeps the eyes wet, thereby preventing the adhesion of the eyelid and the conjunctiva, and also maintains the physiological states of the cornea and the conjunctiva. In corneal epithelium, cells are columnar at the basal section but become flatter toward the surface. The epithelial cells are divided at the basal section gradually migrate upwards to finally be shed off and carried away by tears. The corneal endothelial cells will not be regenerated because they do not undergo cell division. The delayed treatment or chronic state of corneal/conjunctival diseases resulted from corneal disorders such as dry eye, corneal ulcer, corneal erosion and keratitis damages the structures and functions of not only epithelium but also stroma and endothelium, and seriously impairs vision and barrier function. The corneal/conjunctival diseases including a repeated erosion of the cornea and a prolonged corneal epithelial deficiency are the disorders of such. The repairing process of the corneal/conjunctival epithelial disorders involves the coverage of the epithelial deficiency by the migration of cornea epithelial cells, followed by a subsequent cell division and differentiation, resulting in reconstitution of normal cornea and conjunctiva. The factors involved in repair of the cornea/conjunctiva epithelium are being reported (see, e.g., Non-patent Reference 1).

For example, the dry eyes due to a series of disorders such as Sjoegren's syndrome, Stevens Johnson syndrome, meibomian gland function insufficiency and VDT (visual display terminal) syndrome, or ophthalmological operations (e.g., cataract surgery, keratoplasty, refractive surgery); keratoconjunctivitis sicca; and the corneal disorders such as superficial punctate keratopathy (SPK) i.e. a fine spot-like multiple epithelial deficiency occurs on the cornea epithelium observed in drug-induced corneal epithelial disorders, neuroparalytic keratitis, diabetic keratopathy, allergic conjunctivitis, etc.; also seriously impair vision and the barrier functions (see, e.g., Non-Patent Reference 2). Further, the outbreaks of corneal epithelial damages such as superficial punctate keratopathy (SPK) among contact lens wearers have become a big problem in recent years (see, e.g., Non-Patent Reference 3).

As components of therapeutic agents for the corneal epithelial wound, such as fibronectin, EGF (epidermal growth factor) and hyaluronic acid are included. Fibronectin, being a blood-product purified from the patient's own blood by use of a specific purification kit, is not fully utilized in clinical practice because the preparation thereof is time and labor consuming and gives much burden to a patient. EGF, although induces proliferation of corneal epithelial cells, is hardly used in clinical practice because it may cause a critical side effect such as neovascularization when used for inflammatory condition or diabetic keratopathy.
Hyaluronate is glucosamine glycan with a molecular weight of several millions having N-acetyl-D-glucosamine and D-glucuronic acid as constituting saccharides and is used for dry eye patients for its water-retention effect. Hyaluronate, although effective on adhesion and migration of corneal epithelial cells, only exhibits weak effect on proliferation of the corneal epithelial cell. And what is worse is an increase in viscosity at a higher concentration.

On the other hand, selenoprotein P was confirmed in 1977 as a selenium-containing protein which is different from glutathione peroxidase, and in 1982, it was made clear that selenium is incorporated in the form of selenocystein. Furthermore, in 1991, the full-length amino acid sequence was clarified by the cDNA cloning of rat selenoprotein P, suggesting that selenoprotein P may contain maximum of 10 selenocysteins (see, e.g., Non-Patent Reference 4). It is considered that selenoprotein P plays a role of selenium transporter that transport and supply selenium, which is an essential micronutrient, to the various tissues including the brain (see, e.g., Non-Patent Reference 5). Also, glutathione peroxidase activity that reduce lipid peroxide in the presence of thiols such as glutathione (see, e.g., Non-Patent Reference 6), and peroxynitrite scavenging activity (see, e.g., Non-Patent Reference 7) have been reported. In recent years, it has been found that selenoprotein P and the fragment thereof have a cell-death inhibitory activity (see, e.g., Patent Reference 1); a survival-promoting activity in primary nerve cell culture (see, e.g., Non-Patent Reference 8); an activity to improve abnormalities in neurotransmitter function due to the promotion of the synapse formation (see, e.g., Patent Reference 2); an activity to improve inflammation disorder represented by the inhibitory effect on interleukin 6 production (see, e.g., Patent Reference 3); and an activity to improve neurodegeneration due to the improvement of ataxia (see, e.g., Patent Reference 4).
However, the use of selenoprotein P for the corneal/conjunctival disease is not described or suggested in any of the above documents. Moreover, it is difficult to predict the effects of selenoprotein P for the ophthalmologic disorders described herein, and indeed, no such an activity has been reported.

Non-Patent Reference 1: Suzuki K et al, Progress in Retinal and Eye Research, 22, 113 to 133 (2003)
Non-Patent Reference 2: Oh'hashi Y, "2. Superficial punctate keratopathy", "Corneal Clinic, 2nd Edition", p. 36 - 43 (2003), Edited by Manabe R., Kinoshita S., and Oh'hashi Y., Published by Igaku Shoin
Non-Patent Reference 3: Hamano, H., J. Jpn. CL Soc., 37, 1- 6 (1995)
Non-Patent Reference 4: Hill K. E. and Burk R. P., Biomed. Environ. Sci., 10, p. 198 - 208 (1997)
Non-Patent Reference 5: Richardson D. R. Biochem. J. 386, e5 - e7 (2005)
Non-Patent Reference 6: Saito Y et al. J. Biol. Chem. 274, 2866 - 2871 (1999)
Non-Patent Reference 7: Arteel G. E. et al. Biol. Chem. 379, 1201-1205 (1998)
Non-Patent Reference 8: Yan J and Barrentt J. N., J. Neurosci., 18, 8682 - 8691 (1998)
Patent Reference 1: International. Laid Open Patent WO00/31131 Brochure
Patent Reference 2: Laid Open Patent Gazette, Laid Open Patent Publication No. 2004 - 182616
Patent Reference 3: Laid Open Patent Gazette, Laid Open Patent Publication No. 2004 - 182683
Patent Reference 4: International. Laid Open Patent WO02/92121 Brochure

### Disclosure of the Invention

### Problem to be Solved by the Invention

As described above, no conventional composition satisfactory to treat a corneal/conjunctival disease having been known, an excellent composition therefor is desired. Also, more significant composition for prophylactic or therapeutic agents directed to ophthalmologic disorders such as dry eye, keratoconjunctivitis sicca, superficial punctate keratopathy, corneal erosion and corneal ulcer is desired.
The present invention is to provide a novel composition to treat a corneal/conjunctival disease.

### Means for Solving the Problems

As a result of screening of biological compositions of which therapeutic effects on a corneal/conjunctival disease have not been reviewed so far, the inventors of the present invention have found that selenoprotein P present in human blood has excellent therapeutic effects on a corneal/conjunctival disease.

That is to say, the present invention is to provide a prophylactic or therapeutic agent for a corneal/conjunctival disease containing selenoprotein P as an active ingredient. In more detail, the present invention is to provide a prophylactic or therapeutic agent containing selenoprotein P as an active ingredient for a corneal/conjunctival disease such as dry eye, keratoconjunctivitis sicca, superficial punctate keratopathy, corneal erosion and corneal ulcer, or preferably a prophylactic or therapeutic agent for a corneal/conjunctival disease such as dry eye, keratoconjunctivitis sicca, superficial punctate keratopathy, corneal erosion and corneal ulcer, accompanied by corneal/conjunctival epitherial disorders.

In addition, the present invention is to provide a prophylactic or therapeutic agent containing selenoprotein P as an active ingredient for dry eye, keratoconjunctivitis sicca, superficial punctate keratopathy, corneal erosion and corneal ulcer, or preferably a prophylactic or therapeutic agent for dry eye, keratoconjunctivitis sicca, superficial punctate keratopathy, corneal erosion and corneal ulcer, accompanied by corneal/conjunctival epitherial disorders.

Further, the present invention is to provide an eye drop or an eye ointment by which to prevent or treat a corneal/conjunctival disease such as dry eye, keratoconjunctivitis sicca, superficial punctate keratopathy, corneal erosion and corneal ulcer, or preferably an eye drop or an eye ointment by which to prevent or treat a corneal/conjunctival disease such as dry eye, keratoconjunctivitis sicca, superficial punctate keratopathy, corneal erosion and corneal ulcer accompanied by corneal/conjunctival epitherial disorders.
The present invention relates to a composition for ophthalmology containing selenoprotein P, or fragments thereof, and an ophthalmologically acceptable carrier of an ophthalmological agent for the prevention or treatment of a corneal/conjunctival disease such as dry eye, keratoconjunctivitis sicca, superficial punctate keratopathy, corneal erosion and corneal ulcer.
The present invention relates to the use of selenoprotein P or fragments thereof as an active ingredient for a prophylactic or therapeutic agent for a corneal/conjunctival disease.
The present invention further relates to the use of selenoprotein P or fragments thereof for the production of a prophylactic or therapeutic agent for a corneal/conjunctival disease.
Moreover, the present invention relates to an eye drop or an eye ointment by which to prevent or treat a corneal/conjunctival disease such as dry eye, keratoconjunctivitis sicca, superficial punctate keratopathy, corneal erosion and corneal ulcer, comprising administrating an effective amount of selenoprotein P or fragments thereof to a patient suffering from or at a risk of a corneal/conjunctival disease such as dry eye, keratoconjunctivitis sicca, superficial punctate keratopathy, corneal erosion and corneal ulcer, or preferably to a patient suffering from or at a risk of dry eye, keratoconjunctivitis sicca, superficial punctate keratopathy, corneal erosion, corneal ulcer or the like accompanied by corneal/conjunctival epitherial disorders.
Preferably, the present invention relates to a method for preventing/treating dry eye, keratoconjunctivitis sicca, superficial punctate keratopathy, corneal erosion, corneal ulcer, or the like accompanied by corneal/conjunctival epitherial disorders.

### Effects of the Invention

According to the present invention, it is possible to provide an excellent prophylactic or therapeutic agent for a corneal/conjunctival disease such as dry eye, keratoconjunctivitis sicca, superficial punctate keratopathy, corneal erosion and corneal ulcer, particularly for said disorders accompanied by corneal/conjunctival epitherial disorders.

### The Best Mode for Caring Out the Invention

The selenoprotein P used for the present invention may be a naturally occurring or recombinant protein (see, e.g., JP, 2004-337090, A), preferably is a human selenoprotein P. A naturally occurring human selenoprotein P can be purified from human plasma as described in Examples infra. Selenoprotein P of the present invention can be a full length or a fragment consisting of a partial sequence thereof (see, e.g., WO00/031131).

An excellent therapeutic effect of a selenoprotein P of the present invention has been exhibited on a rat corneal epithelial disorder model as demonstrated in Examples infra. Accordingly, the medicament comprising the same is effective and useful as a prophylactic or therapeutic agent for disorders due to inflammation or deficiency in the conjunctiva and the cornea, for example, a corneal/conjunctival disease, more specifically, dry eye, keratoconjunctivitis sicca, superficial punctate keratopathy, corneal erosion and corneal ulcer, etc., particularly as a prophylactic or therapeutic agent of these disorders accompanied by corneal/conjunctival epithelial disorders.
"Cornea/conjunctiva" herein means cornea and/ or conjunctiva, and "a corneal/conjunctival disease" is a disorder of the cornea and/ or the conjunctiva. In the present invention, the disorders such as dry eye, keratoconjunctivitis sicca, superficial punctate keratopathy, corneal erosion and corneal ulcer are generally be categorized in corneal/conjunctival diseases, however, they are not necessarily be originated from a corneal/conjunctival disease. Therefore, the disorders such as dry eye, keratoconjunctivitis sicca, superficial punctate keratopathy, corneal erosion and corneal ulcer of the present invention encompass those which are not originated from a corneal/conjunctival disease.
"Ophthalmologically acceptable carrier of an ophthalmological agent" is a carrier which can be used for the preparation of the ophthalmological agents such as an eye drop and an eye ointment. The carrier used for ophthalmological agents, being applicable to the specific organ i.e. eye, can be distinguished from those used for the ordinary pharmaceutical compositions (preparations) e.g. oral preparations such as a tablet; percutaneous preparations such as an adhesive patch; parental preparations for blood vessels and muscles such as injections; or the like. Accordingly, the ophthalmic composition of the present invention can be distinguished form an ordinary pharmaceutical composition which comprises selenoprotein P.

The prophylactic or therapeutic agent of the present invention is a pharmaceutical composition comprising selenoprotein P or fragments thereof as an active ingredient and a pharmaceutically acceptable carrier. The pharmaceutical composition may comprise active ingredient(s) other than selenoprotein P or fragments thereof.
The prophylactic or therapeutic agent used for the present invention can be formulated into various forms of ophthalmological preparations known in the art. The agent is preferably used for ophthalmological preparations, particularly for eye drops, and such eye drops can be a water-based eye drop, a non-aqueous eye drop, a suspension eye drop, an emulsified eye drop, an eye ointment, or the like. The agent can be produced by the method known to those skilled in the art, wherein the composition is suitably formulated into a dosage form by combining, if necessary, with the pharmaceutically acceptable carrier such as an isotonic agent, a chelating agent, a stabilizing agent, a pH adjusting agent, a preservative, an antioxidant, a solubilization agent and a viscosity increasing agent.

The isotonic agent includes sugars such as glucose, trehalose, lactose, fructose, mannitol, xylitol, and sorbitol; a multivalent alcohols such as glycerine, polyethylene glycol, and propylene glycol; inorganic salts such as sodium chloride, potassium chloride, and calcium chloride; and preferably is present in an amount ranging from 0% to 5% by weight to the total amount of the composition.

The chelating agent includes an edetate such as disodium edetate, calcium disodium edetate, trisodium edetate, tetrasodium edetate, calcium edetate, ethylene diamine tetraacetate, nitrilo triacetic acid or a salt thereof, sodium hexametaphosphate, citric acid, etc., and preferably is present in an amount ranging from 0% to 0.2% by weight to the total amount of the composition.

The stabilizing agent includes sodium hydrogen sulfite, etc., and preferably is present in an amount ranging from 0 to 1% by weight to the total amount of the composition.

The pH adjusting agent includes an acid such as hydrochloric acid, carbonic acid, acetic acid and citric acid, and further includes an alkali metal hydroxide such as sodium hydroxide and potassium hydroxide; an alkali metal carbonate such as sodium carbonate or a hydrogen carbonate; an alkali metal acetate such as sodium acetate, an alkali metal citrate such as sodium citrate, a base such as trometamol, etc., and preferably is present in an amount ranging from 0% to 20% by weight to the total amount of the composition.

The preservative includes sorbic acid, potassium sorbate, a paraoxy benzoic acid ester such as paraoxy methyl benzoate, paraoxy ethyl benzoate, paraoxy propyl benzoate, and paraoxy butyl benzoate, a quaternary ammonium salt such as chlorhexidine gluconate, benzalkonium chloride, benzethonium chloride, and cetylpyridinium chloride, alkyl polyamino ethyl glycine, chlorobutanol, polyquad, polyhexamethylene biguanide, chlorhexidine, etc., and preferably is present in an amount ranging from 0% to 0.2% by weight to the total amount of the composition.

The antioxidant includes sodium hydrogen sulfite, dry sodium sulfite, sodium pyrosulfite, concentrated mixed tocopherol, etc., and preferably is present in an amount ranging from 0% to 0.4% by weight to the total amount of the composition.

The solubilization agent includes sodium benzoate, glycerine, D-sorbitol, grape sugar, propylene glycol, hydroxyl propyl methyl cellulose, polyvinyl pyroridone, macrogol, D-mannitol, etc., and preferably is present in an amount ranging from 0% to 3% by weight to the total amount of the composition.

The viscosity increasing agent includes polyethylene glycol, methyl cellulose, ethyl cellulose, sodium carmelose, xanthan gum, chondroitin sodium sulfate, hydroxyl ethyl cellulose, hydroxyl propyl cellulose, hydroxyl propyl methyl cellulose, polyvinyl pyrrolidone, polyvinyl alcohol, etc., and preferably is present in an amount ranging from 0% to 70% by weight to the total amount of the composition.

An eye drop can be prepared by dissolving or suspending a desirable component described above in an aqueous solvent such as sterilized purified water and a saline solution, or a non-aqueous solvent such as a vegetable oil such as cotton seed oil, soy bean oil, sesame seed oil and peanut oil, followed by adjusting to a certain osmotic pressure and sterilizing by such as filtration sterilization. When preparing an eye ointment, an ointment base can be included in addition to the various types of components described above. The ointment bases preferably include, but are not limited to, an oil-based base agent such as vaseline, fluid paraffin, and polyethylene; an emulsion base wherein an oil phase and an aqueous phase are emulsified with a surfactant; and a water soluble base agent such as hydroxyl propyl methyl cellulose, carboxyl methyl cellulose, polyethylene glycol, or the like.

When administrating the prophylactic or therapeutic agent of the present invention to a subject suffering from a corneal/conjunctival disease, the dosage may vary depending on the body weight, age, sex, conditions of a patient, dosage form, frequency of administration, etc.. The dose of selenoprotein P for adult human per day ranges from 0.25 to 3000 µg, preferably 2.5 to 300 µg that can be administered once or several times a day. In the case of ophthalmic solutions, it is preferably applied topically to the eye in one to several drops each time.

### Brief Description of the Drawings

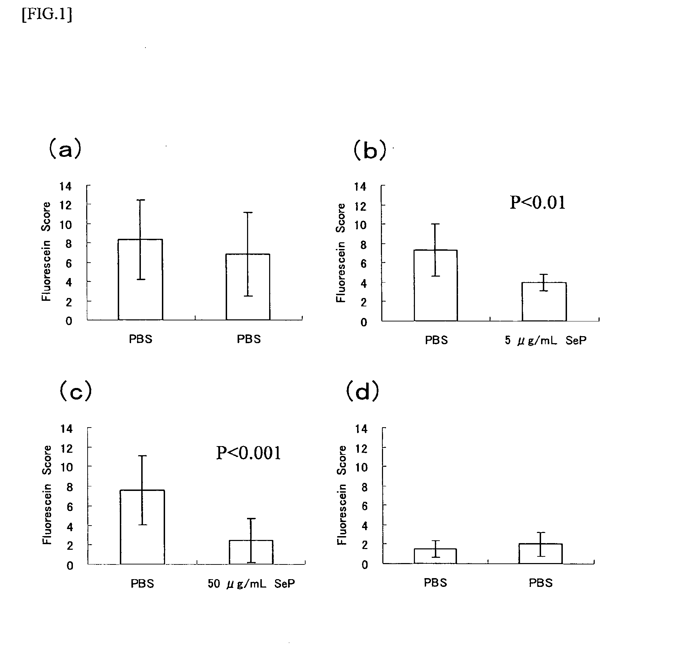

[Fig. 1] The subjects (animal model) were tested in both eyes for the prophylactic activity of selenoprotein P of the present invention against the corneal epithelium disorders. The results were shown in Fig. 1. Fig. 1 (a) shows the results in a control group, Fig. 1 (b) shows the results in the subjects administered with 5 µg/mL of selenoprotein P (PBS solution), Fig. 1 (c) shows the results in the subjects administered with 50 µg/mL of selenoprotein P (PBS solution), and Fig. 1 (d) shows the results in the normal subjects. On each graph, the vertical axis represents the fluorescent score, the left bar shows the results in the control left eyes and the right bar shows the results in the right eyes whereinto the agents were instilled (Fig. 1 (b) and (c)).

The following examples are illustrative of the present invention and should not be construed as limiting the scope of the invention in any manner.

### Example 1

### Preparation of Selenoprotein P

Selenoprotein P was purified from plasma essentially as described by Saito Y. et al., J. Biol. Chem., Vol. 274, p. 2866 to 2871, (1999).
Two litters of frozen fresh human plasma was thawed completely in a warm water bath, and placed in a low temperature chamber. To this was gradually added 100 g of PEG 4000 by a small quantity each time under stirring. The mixture was further stirred for 1 hour after the addition of the total quantity and centrifuged for 20 minutes at 10,000 x g. The supernatant was collected, and filtered through AP25 (Millipore). A column filled with 100 mL of heparin sepharose (Amersham Pharmacia Biotech) was equilibriated in advance with 20 mM phosphate buffer (20 mM phosphoric acid (pH 7.4), 0.15 M NaCl, and 0.2 mM EDTA) and the whole filtrate obtained was applied to the column. After washing the column with 20-fold volume of equilibrium buffer, the adsorbed proteins were eluted with a linear gradient of salt from 0.15 M to 0.6 M. The elution was fractionated into 5 mL each, selenoprotein P in each fraction was quantitated by ELISA, and the fractions containing selenoprotein P were pooled. DFP (diisopropyl fluorophosphates) was added thereto to make the final concentration of 5 mM, in order to avoid the fragmentation of selenoprotein P during the purification process.
The fractions were further diluted 6 times with 20 mM Tris buffer (20 mM Tris - HCl (pH 8)) to make the total volume of 0.75 to 1 L. The solution was applied to 40 mL of Q Sepharose (Amersham Pharmacia Biotech) column pre-equilibrated with 20 mM Tris buffer. The column was washed with 250 to 300 mL of the equilibrium buffer, and eluted with a linear gradient of salt up to 0.25 M. 250 mL of the buffer was used for the gradient. Selenoprotein P in each fraction was quantitated by ELISA, and the fractions containing selenoprotein P were pooled. DFP was added thereinto to make the final concentration of 5 mM.
Imidazole was added to the pooled fraction to make the final concentration of 2 mM, and the fraction was applied to 4 mL of Ni - NTA agarose gel (Qiagen) column pre-equilibrated with 20 mM Tris buffer (20 mM Tris - HCl (pH 8), 2 mM imidazole, 1 M NaCl). After application of the fraction, the column was washed with 30 mL of a washing buffer (20 mM Tris - HCl (pH 8), 20 mM imidazole, 1 M NaCl), and then eluted with an elution buffer (20 mM Tris - HCl (pH 8), 150 mM imidazole, 1 M NaCl). The elution was fractionated into 1 mL each, and the fractions with absorbance peak at 280 nm were pooled. The pooled fraction was concentrated to 2.5 mL by centrifugation using SpeedVac (SAVANT), whereto was added DFP to make the final concentration of 5 mM. Finally, the concentrated selenoprotein P fraction was applied to a PD-10 column (Amersham Pharmacia Biotech) equilibrated with a phosphate buffer for desalting and buffer exchange. The selenoprotein P thus obtained migrated at a position corresponding to a molecular weight of about 67,000 on SDS-PAGE, and the most thereof appeared to be full-length selenoprotein P.

### Example 2

### Therapeutic effects test on corneal/conjunctival epithelial disorders

According to the method of Fujihara et al. (Invest. Ophthalmol. Vis. Sci. 42, 96 -100, 2001), the animal model for corneal/conjunctival epithelial disorder caused by dry eye was prepared as shown below, and the curative effects of selenoprotein P on the corneal/conjunctival epithelial disorder were evaluated.
Using both eyes of the animal for the experiment: one eye was used for control and to the other eye was instilled a drug solution, it was enabled to make the comparison between both eyes within the same individual.

### (Experimental method)

Seven-week-old male Sprague-Dawley rats were anesthetized with pentobarbital (35 mg/kg intraperitonealy) and the extraorbital lacrimal glands of both eyes were removed. And then the rats were used as the dry eye models.

From the following day after extraction of the extraorbital lacrimal glands, phosphate buffered saline (PBS) was instilled into the left eyes of each group, and simultaneously into the right eyes, PBS (control group), 5 µg/mL of selenoprotein P (as PBS solution) and 50 µg/mL of selenoprotein P (as PBS solution) were instilled respectively, at a doze of 5 µL, 6 times a day for 3 weeks daily. As a normal group, the animals having no extraction of the extraorbital lacrimal glands, PBS was instilled into both eyes. Six to 7 animals were used for each group.
After 3 weeks of instillation, the cornea of the both eyes were stained with a fluorophore, fluorescein.
The corneal epithelial disorder was assessed by dividing the whole cornea into the total of 9 sections i.e. the sections of upper, middle and lower and left, middle and right; the lesions were scored for each section according to the standard as shown below, and the total scores were calculated. The difference in total scores of the left eyes and the right eyes in each group were assessed according to the Student's t- test. For the fair assessment, a series of experiment, from the beginning of instillation of eye drop to the scoring of the corneal epithelial disorder, was perfomed in a blind fashion.

### (Scoring of fluorescein staining of the corneal epithelium)

0 : not stained (no spot-like fluorescent)
1 : week (slight) spot-like fluorescent staining were observed
2 : relatively many spot-like fluorescent staining were observed
3 : strong (dense) spot-like fluorescent staining were observed

### (Results)

The results are shown in Fig. 1. Fig. 1 (a) shows the results in the control group (hereinafter referred to as C group), Fig. 1 (b) shows the results in the group instilled with 5 µg/mL of selenoprotein P (PBS solution) (hereinafter referred to as S5 group), Fig. 1 (c) shows the results in the group instilled with 50 µg/mL of selenoprotein P (PBS solution) (hereinafter referred to as S50 group), and Fig. 1 (d) the results in the normal group (hereinafter referred to as N group).
The vertical axis in the each figure shows the fluorescent score, and the left bar shows the results of the control left eyes instilled with PBS, and the right bar shows the results of the right eyes instilled with PBS ((a) and (d)) or selenoprotein P (denoted with SeP) of 5 µg/mL or 50 µg/mL (PBS solutions) ((b) and (c) respectively). The score values were given in average ± SEM.
The fluorescent scores of the left bar in the graphs ((a) to (c)) indicated between 7.3 to 8.3 in average, revealing the progression of corneal epithelial disorder as a result of 3 weeks of PBS instillation after extraction of the extraorbital lacrimal gland. In the contrast, the fluorescent scores of the N group (with no extraction of the extraorbital lacrimal gland) in Fig 1(d) were 1.5 (left eyes) and 2.0 (right eyes) in average after 3 weeks of PBS instillation.
On the other hand, for C group, the fluorescent score of the right bar in Fig. 1 (a) was 6.8 in avarage, and hardly any change was observed when compared with the scores of the left eyes. In contrast, S5 group in Fig. 1 (b) indicated the average fluorescent scores of 4.0, and S50 group in (c) indicated 2.4, revealing the significant inhibition in progression of the corneal epithelial disorder in a dose dependent manner (the significant differences observed in Fig. 1 (b) at P < 0.01 and in Fig. 1 (c) at P < 0.001). Particularly, it was observed that the average of the fluorescent scores of S50 group in (c) was reduced close to the score ofN group in (d).
It is apparent from the results of the above pharmacological study, that the corneal epithelial disorder confirmed by the fluorescein staining was suppressed by the instillation of selenoprotein P in a dose-dependent manner. Namely, the progression of the corneal epithelial disorder was suppressed by the instillation of selenoprotein P.
Consequently, it has been demonstrated that selenoprotein P is useful as a prophylactic or therapeutic agent for a corneal/conjunctival disease such as dry eye, keratoconjunctivitis sicca, superficial punctate keratopathy, corneal erosion and corneal ulcer, particularly for said disorders accompanied by a corneal/conjunctival epithelial disorder.

### Industrial Applicability

The present invention is to provide a novel pharmaceutical composition for the ophthalmological formulations in use for prevention and treatment of a corneal/conjunctival disease, which is industrially useful.

## Claims

1. A prophylactic or therapeutic agent for a corneal/conjunctival disease comprising selenoprotein P as an active ingredient.

2. The prophylactic or therapeutic agent according to Claim 1, wherein the corneal/conjunctival disease is dry eye, keratoconjunctivitis sicca, superficial punctate keratopathy, corneal erosion or corneal ulcer.

3. The prophylactic or therapeutic agent for dry eye, keratoconjunctivitis sicca, superficial punctate keratopathy, corneal erosion or corneal ulcer, comprising selenoprotein P as an active ingredient.

4. The prophylactic or therapeutic agent according to any one of Claims 1 to 3, wherein said prophylactic or therapeutic agent is an eye drop or an eye ointment.

5. An ophthalmological composition for comprising selenoprotein P and an ophthalmologically acceptable carrier.

6. A use of selenoprotein P for producing the prophylactic or therapeutic agent for the corneal/conjunctival disease.

7. The use according to Claim 6 for producing the prophylactic or therapeutic agent, wherein the corneal/conjunctival disease is dry eye, keratoconjunctivitis sicca, superficial punctate keratopathy, corneal erosion or corneal ulcer.

8. A method of preventing/treating for the corneal/conjunctival disease, comprising administering an effective amount of selenoprotein P to a patient suffering from or at a risk of said corneal/conjunctival disease.

9. The method according to Claim 8, wherein the corneal/conjunctival disease is dry eye, keratoconjunctivitis sicca, superficial punctate keratopathy, corneal erosion or corneal ulcer.
